(19) Europäisches Patentamt
European Patent Office
Office européen des brevets

(11) Veröffentlichungsnummer: **0 272 430**
**A1**

(12) # EUROPÄISCHE PATENTANMELDUNG

(21) Anmeldenummer: **87116506.4**

(22) Anmeldetag: **09.11.87**

(51) Int. Cl.⁴: **C07D 275/06**, C07C 87/28, C07D 215/02, A01N 43/80

(30) Priorität: **22.11.86 DE 3639900**

(43) Veröffentlichungstag der Anmeldung: **29.06.88 Patentblatt 88/26**

(84) Benannte Vertragsstaaten: **BE CH DE FR GB IT LI NL**

(71) Anmelder: **BAYER AG**
**Konzernverwaltung RP Patentabteilung**
**D-5090 Leverkusen 1 Bayerwerk(DE)**

(72) Erfinder: **Weissmüller, Joachim, Dr.**
**Carl-Langhans-Strasse 53**
**D-4019 Monheim(DE)**
Erfinder: **Reinecke, Paul, Dr.**
**Steinstrasse 8**
**D-5090 Leverkusen 3(DE)**
Erfinder: **Hänssler, Gerd, Dr.**
**Am Arenzberg 58a**
**D-5090 Leverkusen 3(DE)**

(54) **Saccharin-Salze von substituierten Aminen.**

(57) Saccharin-Salze von substituierten Aminen der allgemeinen Formel (I),

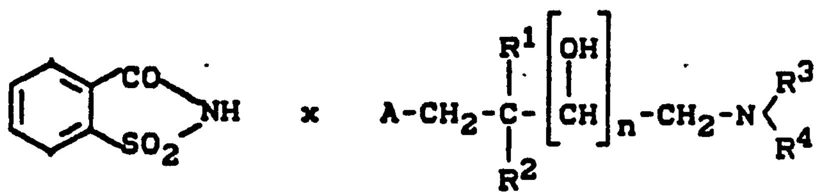
(I)

in welcher
A für jeweils gegebenenfalls substituiertes Cyclohexyl, Cyclohexenyl oder Phenyl steht,
$R^1$ für Wasserstoff oder Alkyl steht,
$R^2$ für Wasserstoff oder Alkyl steht,
$R^3$ und $R^4$ unabhängig voneinander für Alkyl stehen oder gemeinsam mit dem Stickstoffatom, an welches sie gebunden sind, für einen gegebenenfalls substituierten, gesättigten Heterocyclus stehen, der weitere Heteroatome enthalten kann und
n für eine Zahl 0 oder 1 steht,
und ihre Verwendung als Schädlingsbekämpfungsmittel.
Die Verbindungen der Formel (I) können aus geeigneten Aminen und Saccharin hergestellt werden.

EP 0 272 430 A1

Xerox Copy Centre

## Saccharin-Salze von substituierten Aminen

Die Erfindung betrifft neue Saccharin-Salze von substituierten Aminen, ein Verfahren zu ihrer Herstellung sowie ihr Verwendung als Schädlingsbekämpfungsmittel.

Es ist bereits bekannt, daß Saccharin-Salze von Aminen, wie beispielsweise das Saccharin-Salz des 5-Amino-1,2,4-triazols, fungizide Eigenschaften besitzen (vgl. EP 158 074).

Weiterhin ist bekannt, daß bestimmte substituierte Amine und deren Salze, wie beispielsweise das Ameisensäuresalz des 1-(4-t-Butylphenyl)-3-(3,5-dimethylpiperidin-1-yl)-2-methyl-propans, ebenfalls fungizide Eigenschaften besitzen (vgl. DE-OS 2 752 135).

Die Wirkung dieser vorbekannten Verbindungen ist jedoch insbesondere bei niedrigen Aufwandmengen und Konzentrationen nicht in allen Anwendungsgebieten völlig zufriedenstellend.

Es wurden neue Saccharin-Salze von substituierten Aminen der allgemeinen Formel (I),

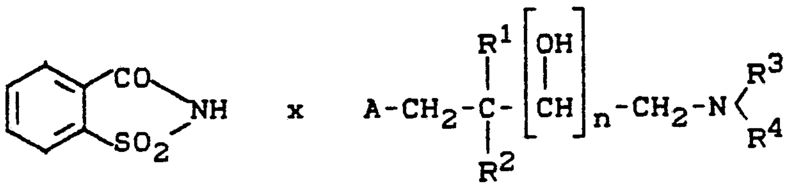

(I)

in welcher

A für jeweils gegebenenfalls substituiertes Cyclohexyl, Cyclohexenyl oder Phenyl steht,

$R^1$ für Wasserstoff oder Alkyl steht,

$R^2$ für Wasserstoff oder Alkyl steht,

$R^3$ und $R^4$ unabhängig voneinander für Alkyl stehen oder gemeinsam mit dem Stickstoffatom, an welches sie gebunden sind, für einen gegebenenfalls substituierten, gesättigten Heterocyclus stehen, der weitere Heteroatome enthalten kann und

n für eine Zahl 0 oder 1 steht,

gefunden.

Die Verbindungen der Formel (I) können als geometrische und/oder optische Isomere oder Isomerengemische unter schiedlicher Zusammensetzung vorliegen. Sowohl die reinen Isomeren als auch die Isomerengemische werden erfindungsgemäß beansprucht.

Weiterhin wurde gefunden, daß man die neuen Saccharin-Salze von substituierten Aminen der allgemeinen Formel (I),

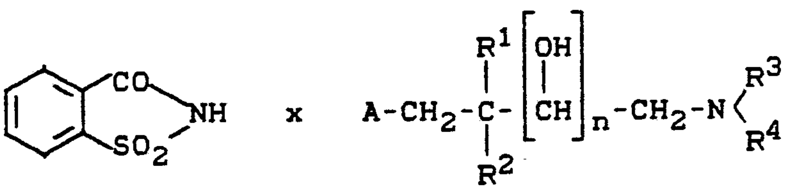

(I)

in welcher

A für jeweils gegebenenfalls substituiertes Cyclohexyl, Cyclohexenyl oder Phenyl steht,

$R^1$ für Wasserstoff oder Alkyl steht,

$R^2$ für Wasserstoff oder Alkyl steht,

$R^3$ und $R^4$ unabhängig voneinander für Alkyl stehen oder gemeinsam mit dem Stickstoffatom, an welches sie gebunden sind, für einen gegebenenfalls substituierten, gesättigten Heterocyclus stehen, der weitere Heteroatome enthalten kann und

n für eine Zahl 0 oder 1 steht,

erhält, wenn man substituierte Amine der Formel (II),

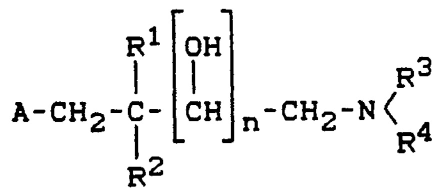 (II)

in welcher

A, $R^1$, $R^2$, $R^3$, $R^4$ und n die oben angegebene Bedeutung haben,

mit Saccharin gegebenenfalls in Gegenwart eines Verdünnungsmittels umsetzt.

Schließlich wurde gefunden, daß die neuen Saccharin-Salze von substituierten Aminen der allgemeinen Formel (I) eine Wirkung gegen Schädlinge besitzen.

Überraschenderweise zeigen die erfindungsgemäßen Saccharin-Salze von substituierten Aminen der allgemeinen Formel (I) u.a. eine bessere fungizide Wirksamkeit als die aus dem Stand der Technik bekannten Saccharin-Salze von Aminen, wie beispielsweise das 5-Amino-1,2,4-triazol oder die aus dem Stand der Technik bekannten substituierten Amine und/oder deren Salze, wie beispielsweise das Ameisensäuresalz des 1-(4-t-Butyl-phenyl)-3-(3,5-dimethylpiperidin-1-yl)-2-methyl-propans, welches chemisch und wirkungsmäßig naheliegende Verbindungen sind.

Die erfindungsgemäßen Saccharin-Salze von substituierten Aminen sind durch die Formel (I) allgemein definiert. Bevorzugt sind Salze der Formel (I), bei welchen

A für jeweils ein-bis dreifach, gleich oder verschieden substituiertes Cyclohexyl oder Cyclohexenyl steht, wobei als Substituenten jeweils infrage kommen: geradkettiges oder verzweigtes Alkyl mit 1 bis 6 Kohlenstoffatomen, Halogenalkyl oder Halogenalkoxy mit jeweils 1 oder 2 Kohlenstoffatomen und 1 bis 5 gleichen oder verschiedenen Halogenatomen; oder für gegebenenfalls ein-bis dreifach, gleich oder verschieden substituiertes Phenyl steht, wobei als Substituenten infrage kommen: Halogen, geradkettiges oder verzweigtes Alkyl mit 1 bis 6 Kohlenstoffatomen, Halogenalkyl oder Halogenalkoxy mit jeweils 1 oder 2 Kohlenstoffatomen und 1 bis 5 gleichen oder verschiedenen Halogenatomen, Alkenyl mit 3 bis 6 Kohlenstoffatomen oder Cycloalkyl mit 3 bis 7 Kohlenstoffatomen,

$R^1$ für Wasserstoff oder für geradkettiges oder verzweigtes Alkyl mit 1 bis 4 Kohlenstoffatomen steht,

$R^2$ für Wasserstoff oder für geradkettiges oder verzweigtes Alkyl mit 1 bis 4 Kohlenstoffatomen steht,

$R^3$ und $R^4$ unabhängig voneinander jeweils für geradket tiges oder verzweigtes Alkyl mit 1 bis 6 Kohlenstoffatomen stehen oder gemeinsam mit dem Stickstoffatom, an welches sie gebunden sind, für einen gegebenenfalls ein-bis fünffach, gleich oder verschieden substituierten gesättigten fünf-bis siebengliedrigen Heterocyclus stehen, der als weitere Heteroatome insbesondere Stickstoff, Sauerstoff oder Schwefel enthalten kann, wobei als Substituenten infrage kommen: Hydroxy, geradkettiges oder verzweigtes Alkyl, Hydroxyalkyl, Acetoxyalkyl oder Propionyloxyalkyl mit jeweils 1 bis 4 Kohlenstoffatomen in den einzelnen Alkylteilen und

n für eine Zahl 0 oder 1 steht.

Besonders bevorzugt sind Salze der Formel (I), bei welchen

A für jeweils ein-oder zweifach, gleich oder verschieden substituiertes Cyclohexyl oder Cyclohexenyl steht, wobei als Substituenten jeweils genannt seien: Methyl, Ethyl, n-oder i-Propyl, n-, i-, s-, t-Butyl, 2-Methyl-but-2-yl, Trifluormethyl und Trifluormethoxy; oder für gegebenenfalls ein-bis dreifach, gleich oder verschieden substituiertes Phenyl steht, wobei als Substituenten genannt seien: Fluor, Chlor, Brom, Methyl, Ethyl, n-oder i-Propyl, n-, i-, s-oder t-Butyl, 2-Methyl-but-2-yl, Tirfluormethyl, Trifluormethoxy, Allyl, n-oder i-Butenyl und Cyclohexyl;

$R^1$ für Wasserstoff oder Methyl steht,

$R^2$ für Wasserstoff oder Methyl steht,

$R^3$ und $R^4$ unabhängig voneinander jeweils für geradkettiges oder verzweigtes Alkyl mit jeweils 1 bis 6 Kohlenstoffatomen stehen, oder gemeinsam mit dem Stickstoffatom, an welches sie gebunden sind, für einen gegebenenfalls ein-bis dreifach, gleich oder verschieden durch Hydroxy, Hydroxymethyl, Methyl, Ethyl, Acetoxymethyl und Propionyloxymethyl substituierten Heterocyclus der Formel

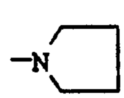 ; 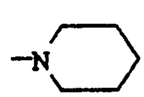 ; 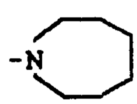 ; 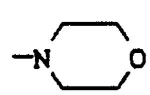 oder 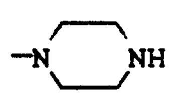

stehen und

n für eine Zahl 0 oder 1 steht.

Ganz besonders bevorzugt sind Salze der Formel (Ia),

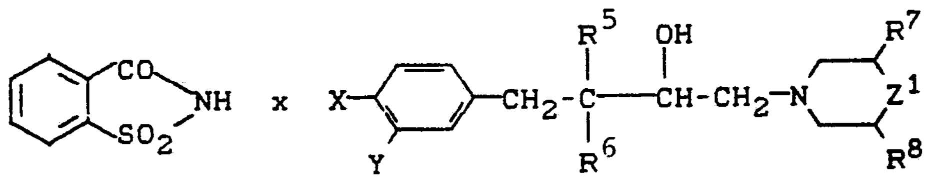 (Ia)

bei welchen
X für Wasserstoff, Fluor, Chlor, i-Propyl oder t-Butyl steht,
Y für Wasserstoff, Fluor, Chlor, Methyl oder Ethyl steht,
$R^5$ für Wasserstoff oder Methyl steht,
$R^6$ für Wasserstoff oder Methyl steht,
$R^7$ und $R^8$ jeweils für Wasserstoff, Methyl oder Ethyl stehen und
$Z^1$ für Sauerstoff oder eine $-CH_2$-Gruppe steht.

Ganz besonders bevorzugt sind außerdem Salze der Formel (Ib),

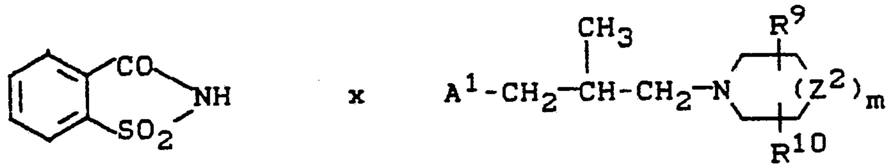 (Ib)

bei welchen
$A^1$ für jeweils ein-oder zweifach, gleich oder verschieden substituiertes Cyclohexyl oder Cyclohexenyl steht, wobei als Substituenten jeweils genannt seien: Methyl, Ethyl, n-oder i-Propyl, n-, i-, s-oder t-Butyl oder 2-Methyl-but-2-yl; oder für ein-oder zweifach, gleich oder verschieden substituiertes Phenyl steht, wobei als Substituenten genannt seien: Fluor, Chlor, Brom, Methyl, Ethyl, n-oder i-Propyl, n-, i-, s-oder t-Butyl, 2-Methyl-but-2-yl oder Cyclohexyl,
$R^9$ und $R^{10}$ unabhängig voneinander für Wasserstoff, Methyl, Ethyl, Hydroxymethyl oder Acetoxymethyl stehen,
$Z^2$ für Sauerstoff oder für eine gegebenenfalls durch Methyl oder Ethyl substituierte Methylen-oder Ethylenguppe steht und
m für eine Zahl 0 oder 1 steht.

Im einzelnen seien die folgenden Salze der allgemeinen Formel (I) genannt:

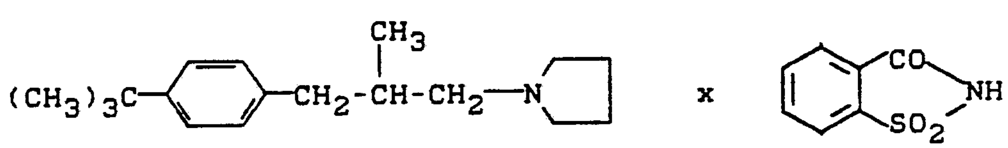

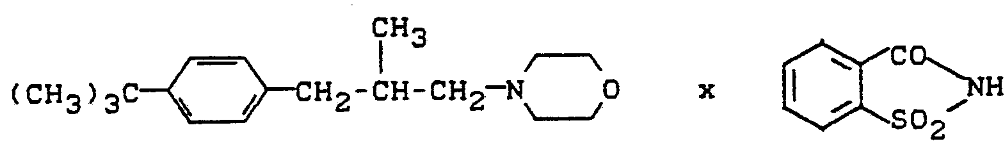

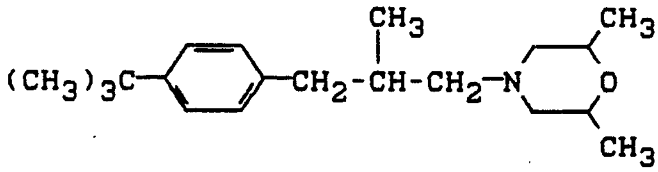 x 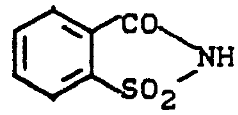

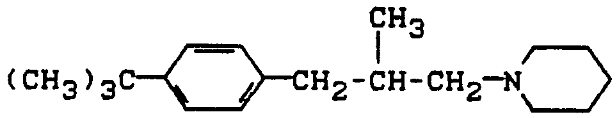 x 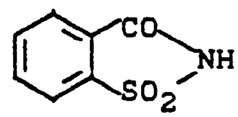

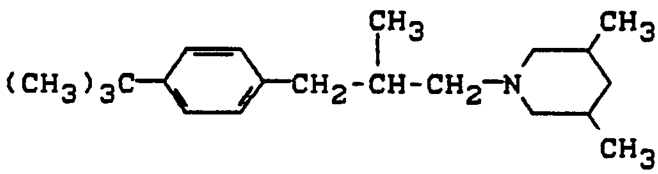 x 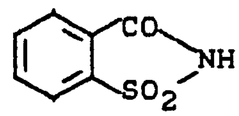

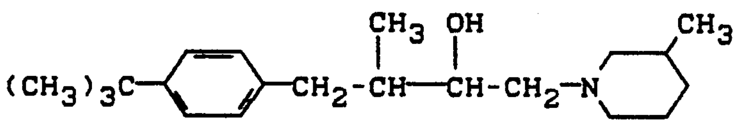 x 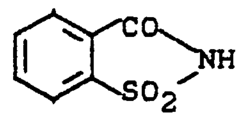

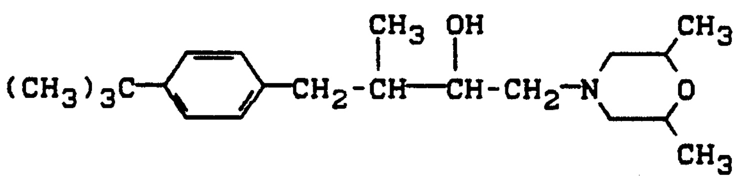 x 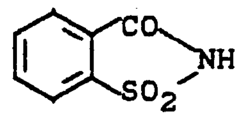

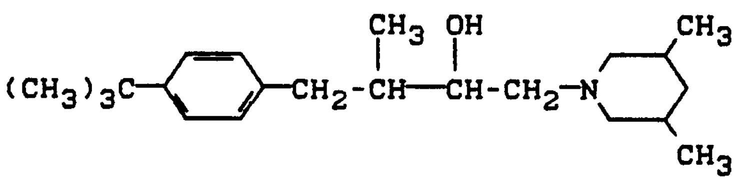 x 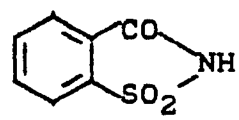

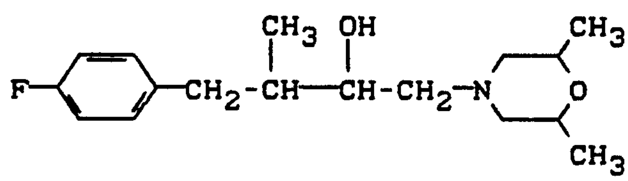 x 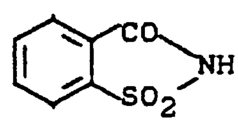

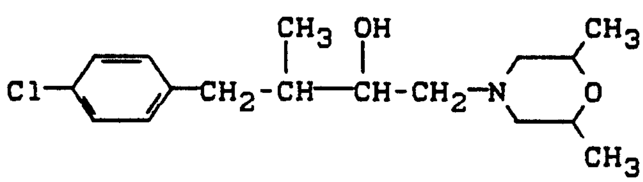 x 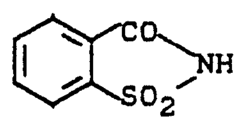

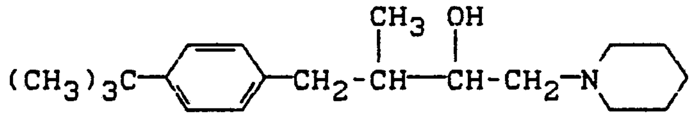 x 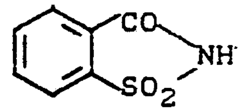

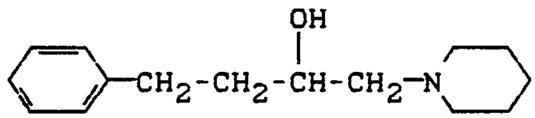 x 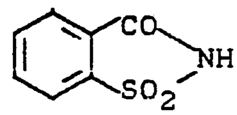

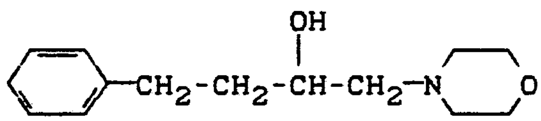 x 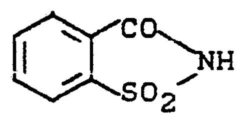

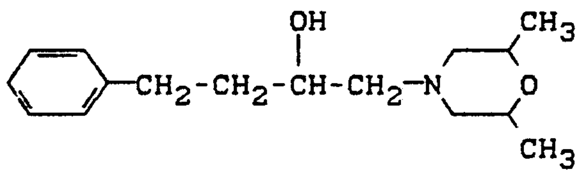 x 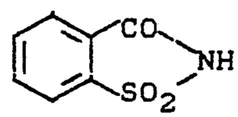

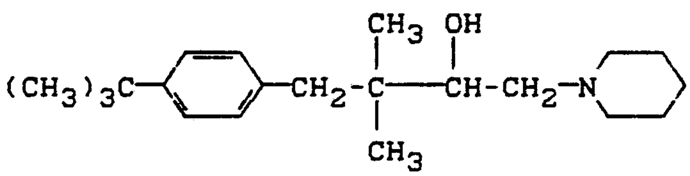 x 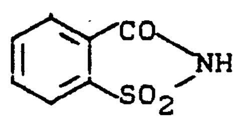

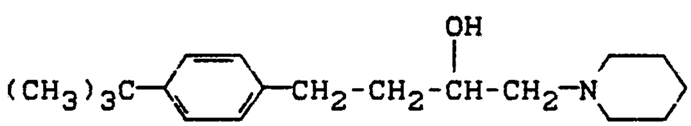 x 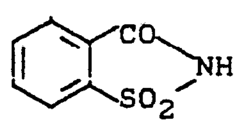

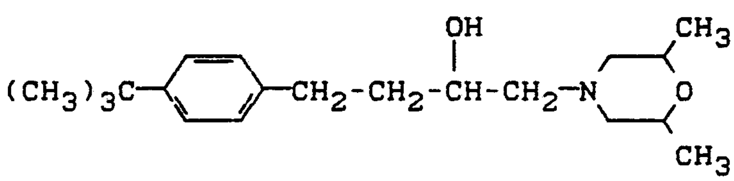 x 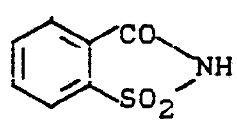

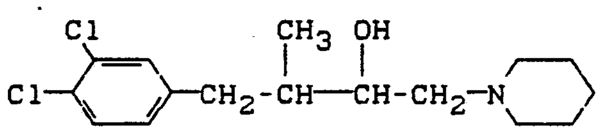 x 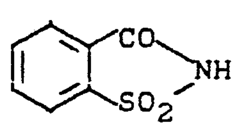

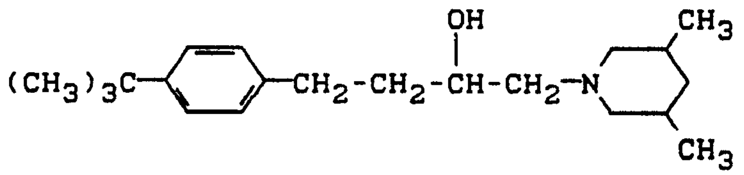 x 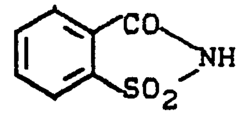

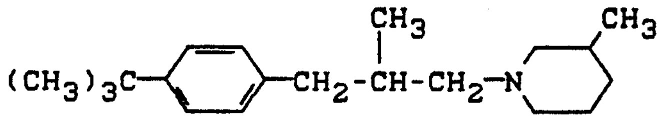 x 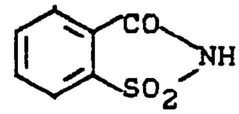

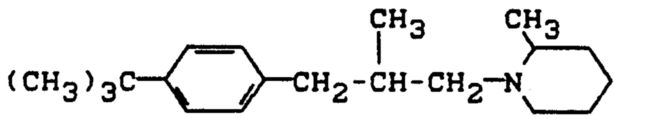 x 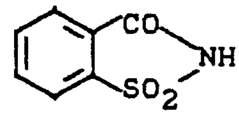

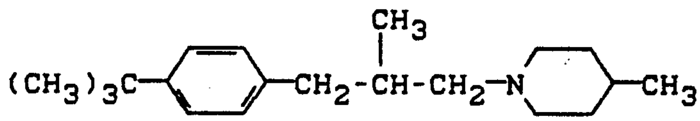 x 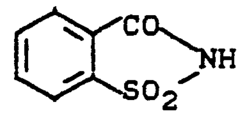

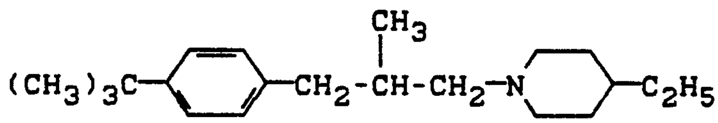 x 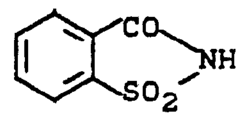

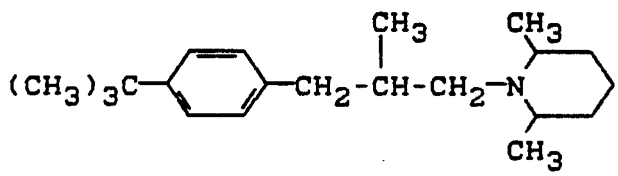 x 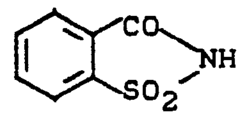

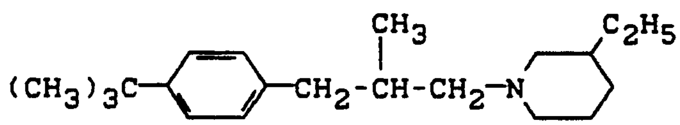 x 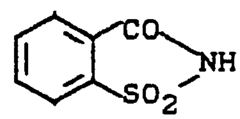

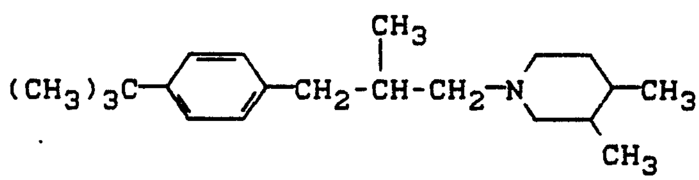 x 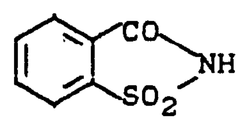

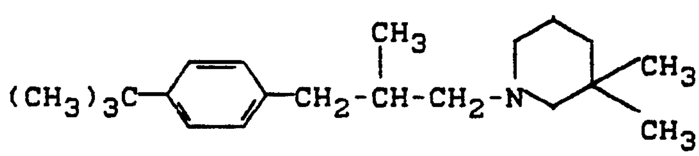 x 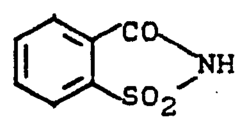

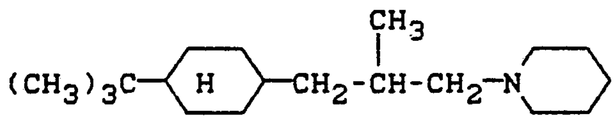 x 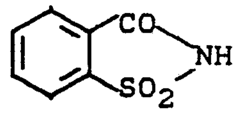

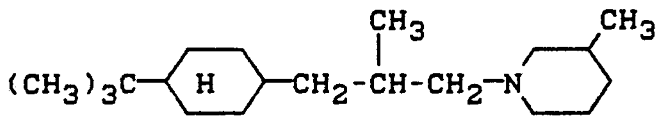 x 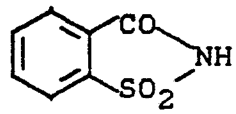

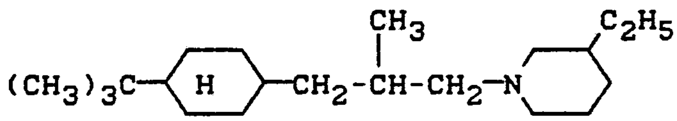 x 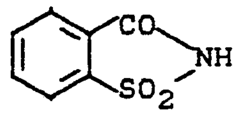

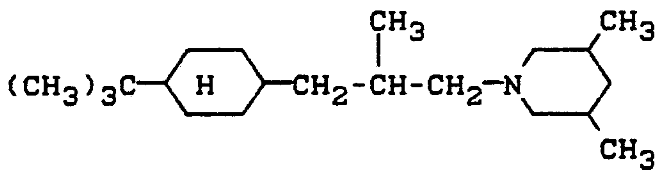 x 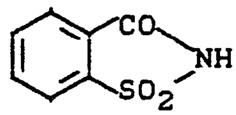

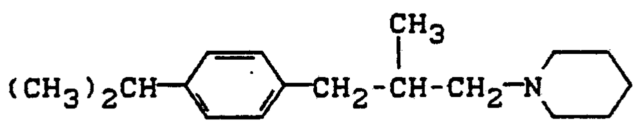 x 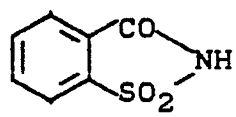

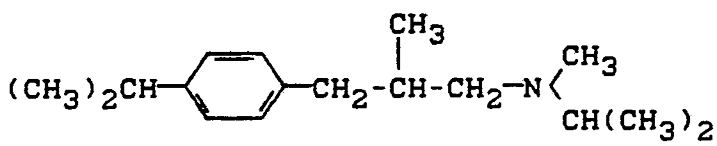 x 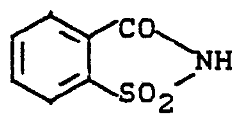

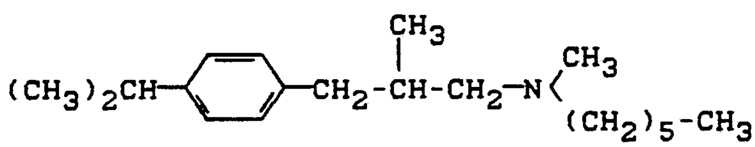 x 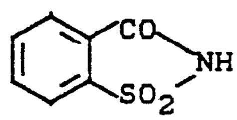

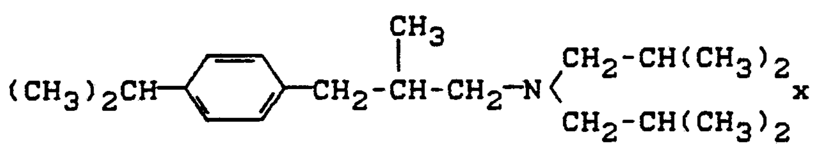 x 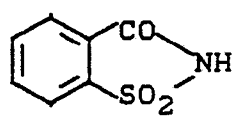

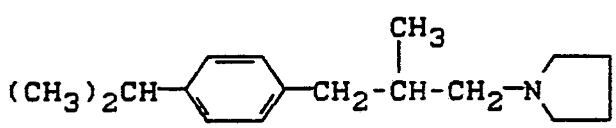 x 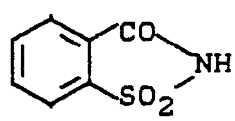

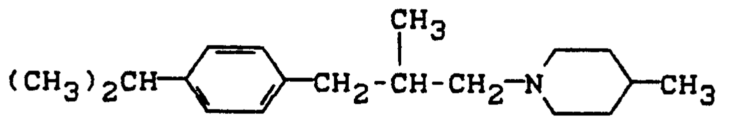 x 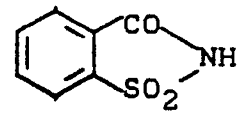

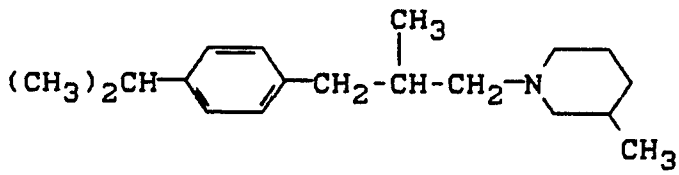 x 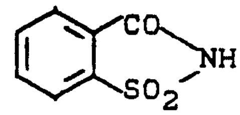

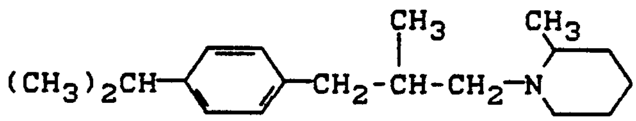 x 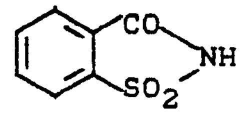

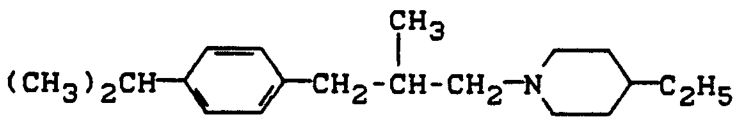 x 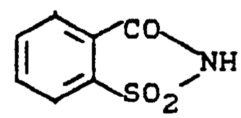

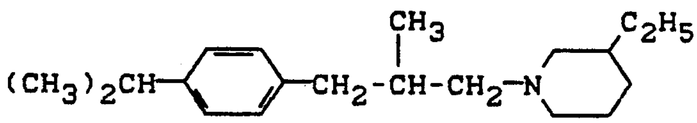 x 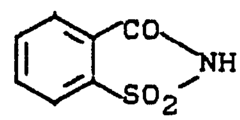

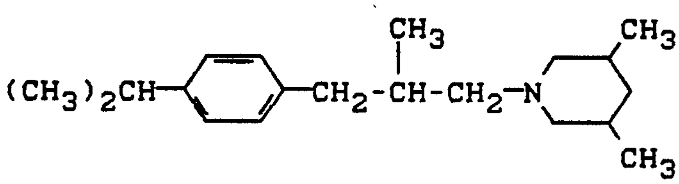 x 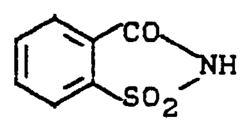

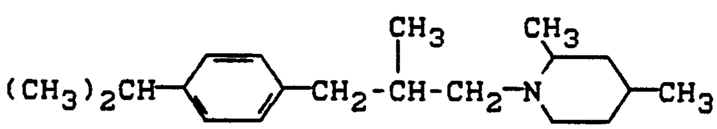 x 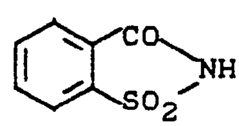

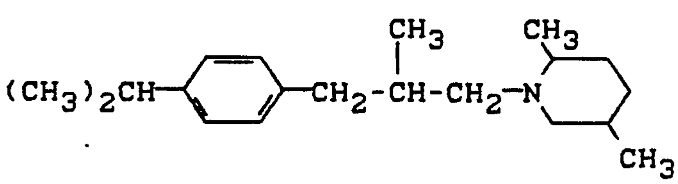 x 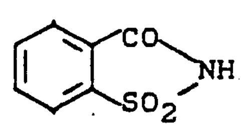

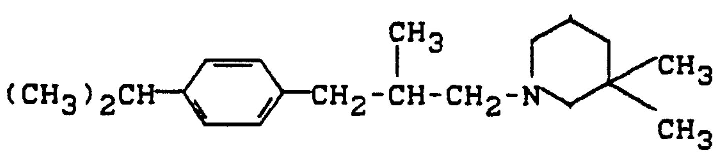 x 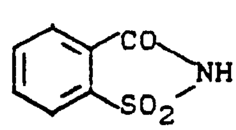

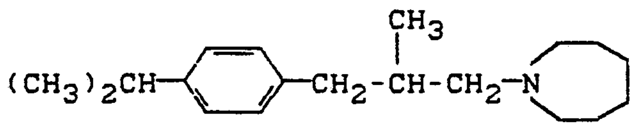 x 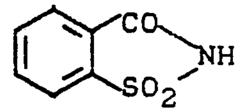

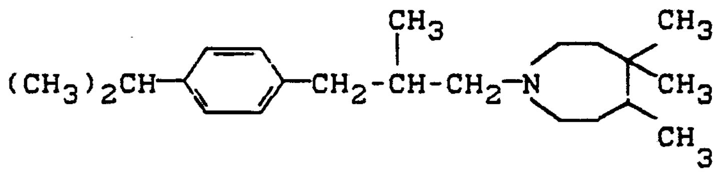 x 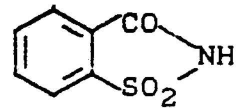

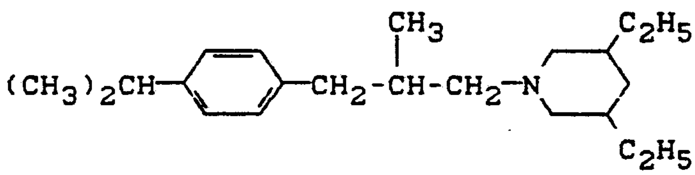 x 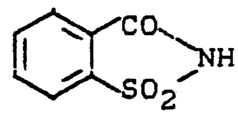

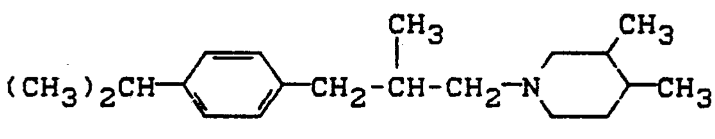 x 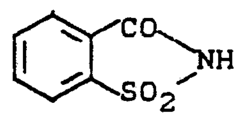

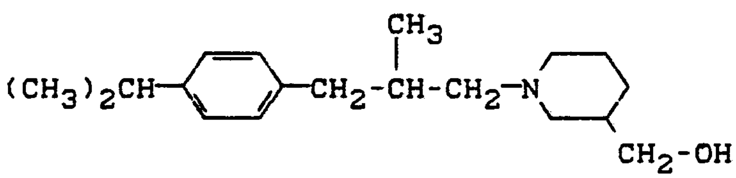 x 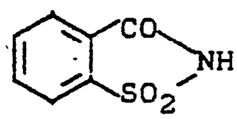

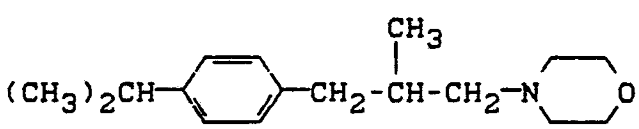 x 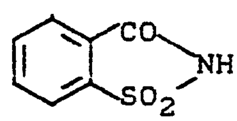

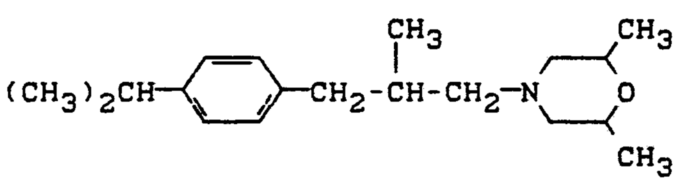 x 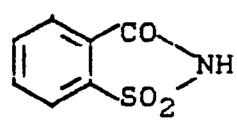

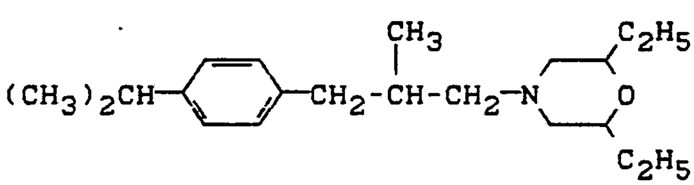 x 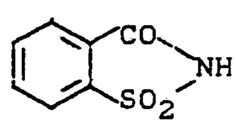

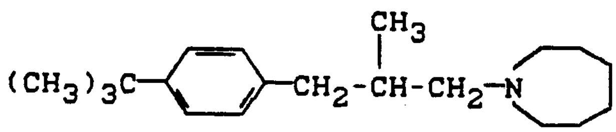 x 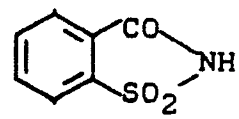

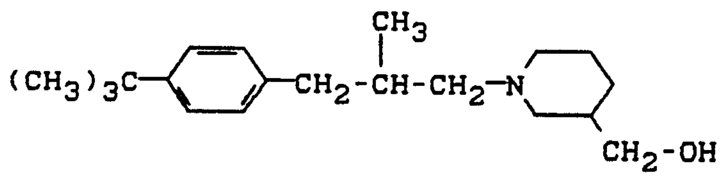 x 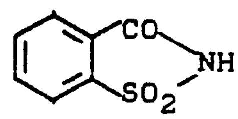

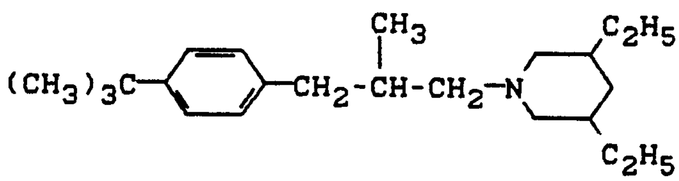 x 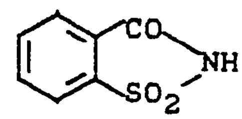

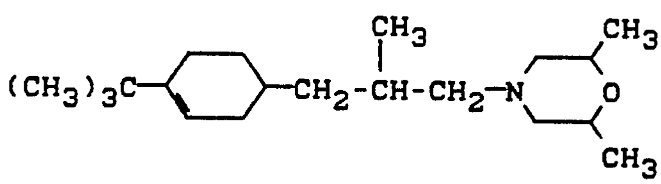 x 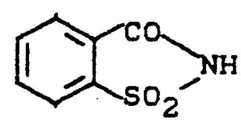

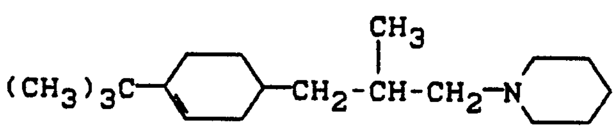 x 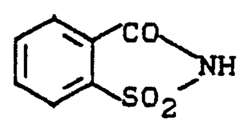

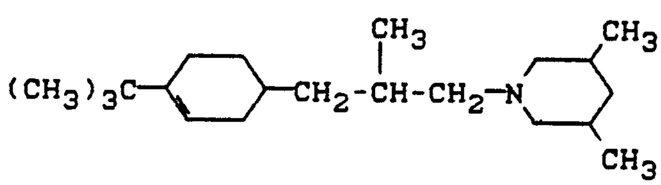 x 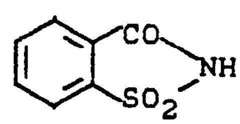

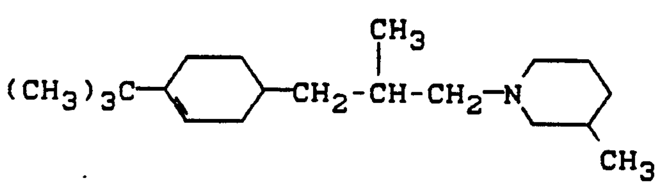 x 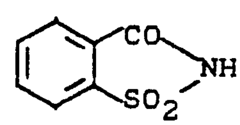

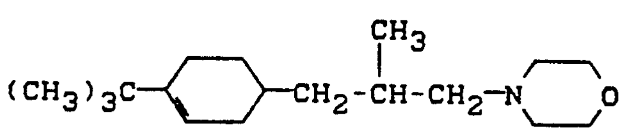 x 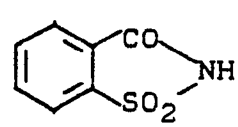

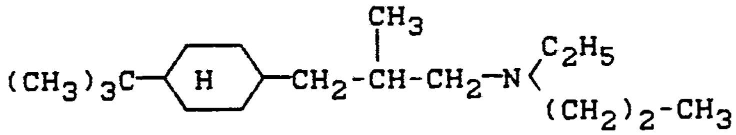 x 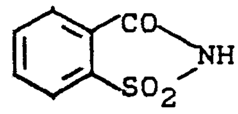

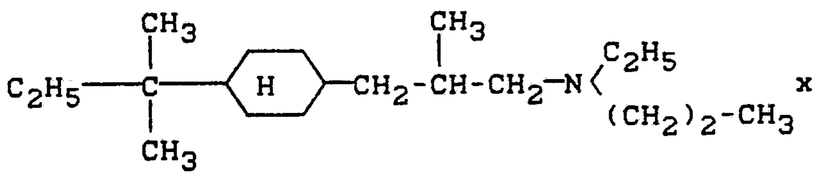 x 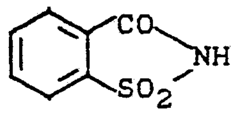

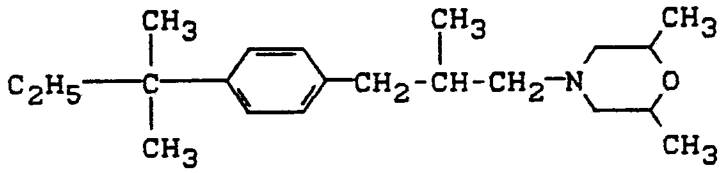 x 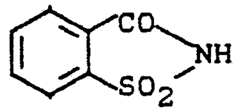

Verwendet man beispielsweise 3-(2,6-Dimethyl-4-morpholinyl)-2-methyl-1-(4-t-butyl-phenyl)-propan und Saccharin als Ausgangsstoffe, so läßt sich der Reaktionsablauf des erfindungsgemäßen Verfahrens durch das folgende Formelschema darstellen:

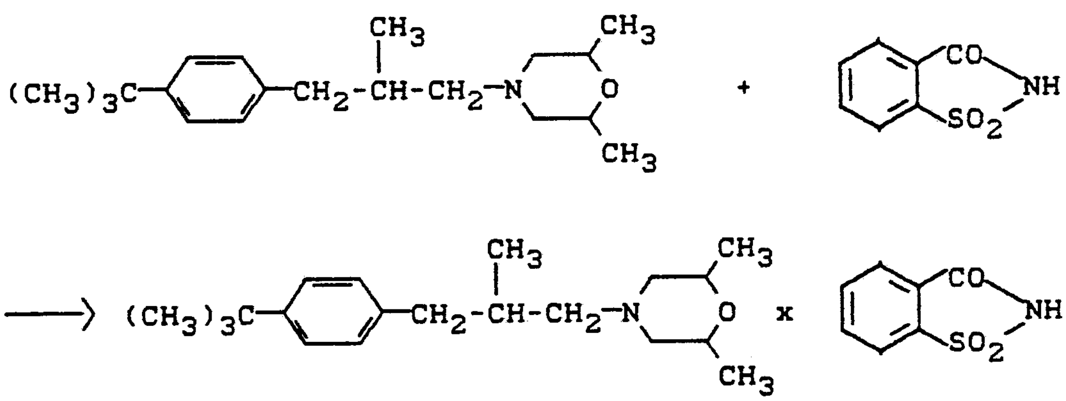

Die zur Durchführung des erfindungsgemäßen Verfahrens als Ausgangsstoffe benötigten substituierten Amine sind durch die Formel (II) allgemein definiert. In dieser Formel (II) stehen A, $R^1$, $R^2$, $R^3$, $R^4$ und n für diejenigen Reste, die bereits im Zusammenhang mit der Beschreibung der erfindungsgemäßen Stoffe der Formel (I) für diese Substituenten genannt wurden.

Die substituierten Amine der Formel (II) sind bekannt (vgl. DE-OS 2 727 482, DE-OS 2 752 135, DE-OS 2 825 961, DE-OS 2 830 127, DE-OS 2 921 131, DE-OS 3 121 349, EP 123 092 und EP 129 321).

Als Verdünnungsmittel zur Durchführung des erfindungsge mäßen Verfahrens kommen inerte organische Lösungsmittel infrage.

Hierzu gehören insbesondere Ether wie Diethylether, Dioxan, Tetrahydrofuran oder Ethylenglykoldimethyl-oder -diethylether; Ketone wie Aceton oder Butanon; Nitrile wie Acetonitril oder Propionitril; Amide wie Dimethylformamid, Dimethylacetamid, N-Methylformanilid, N-Methylpyrrolidon oder Hexamethylphosporsäuretriamid oder Alkohole wie Methanol, Ethanol oder Propanol.

Die Reaktionstemperaturen können bei der Durchführung des erfindungsgemäßen Verfahrens in einem größeren Bereich variiert werden. Im allgemeinen arbeitet man bei Temperaturen zwischen 0 °C und 100 °C, vorzugsweise bei Temperaturen zwischen 10 °C und 50 °C.

Zur Durchführung des erfindungsgemäßen Verfahrens setzt man pro Mol an substituierten Amin der Formel (II) äquimolare Mengen an Saccharin zu. Man löst beide Reaktionspartner bei der geeigneten Reaktionstemperatur in einem geeigneten Lösungsmittel und entfernt anschließend das Lösungsmittel durch Destillation im Vakuum. Die so erhältlichen Salze, die gelegentlich als zähe Öle oder amorph anfallen, können nach allgemein üblichen Verfahren wie z.B. durch Umkristallisieren oder Digerieren in geeigneten Lösungsmitteln gereinigt werden. Die Charakterisierung erfolgt in diesen Fällen mit Hilfe spektroskopischer Verfahren (IR; NMR).

Die erfindungsgemäßen Wirkstoffe weisen eine starke Wirkung gegen Schädlinge auf und können zur Bekämpfung von unerwünschten Schadorganismen praktisch eingesetzt werden. Die Wirkstoffe sind für den Gebrauch als Pflanzenschutzmittel insbesondere als Fungizide geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Beispielhaft aber nicht begrenzend seien einige Erreger von pilzlichen Erkrankungen, die unter die oben aufgezählten Oberbegriffe fallen, genannt:
Pythium-Arten, wie beispielsweise Pythium ultimum;
Phytophthora-Arten, wie beispielsweise Phytophthora infestans;
Pseudoperonospora-Arten, wie beispielsweise Pseudoperonospora humuli oder Pseudoperonospora cubensis;
Plasmopara-Arten, wie beispielsweise Plasmopara viticola;
Peronospora-Arten, wie beispielsweise Peronospora pisi oder P. brassicae;
Erysiphe-Arten, wie beispielsweise Erysiphe graminis; Sphaerotheca-Arten, wie beispielsweise Sphaerotheca fuliginea;
Podosphaera-Arten, wie beispielsweise Podosphaera leucotricha;
Venturia-Arten, wie beispielsweise Venturia inaequalis;
Pyrenophora-Arten, wie beispielsweise Pyrenophora teres oder P. graminea (Konidienform: Drechslera, Syn: Helminthosporium);
Cochliobolus-Arten, wie beispielsweise Cochliobolus sativus (Konidienform: Drechslera, Syn: Helminthosporium);
Uromyces-Arten, wie beispielsweise Uromyces appendiculatus;
Puccinia-Arten, wie beispielsweise Puccinia recondita;
Tilletia-Arten, wie beispielsweise Tilletia caries;
Ustilago-Arten, wie beispielsweise Ustilago nuda oder Ustilago avenae;
Pellicularia-Arten, wie beispielsweise Pellicularia sasakii;
Pyricularia-Arten, wie beispielsweise Pyricularia oryzae;
Fusarium-Arten, wie beispielsweise Fusarium culmorum;
Botrytis-Arten, wie beispielsweise Botrytis cinerea;
Septoria-Arten, wie beispielsweise Septoria nodorum;
Leptosphaeria-Arten, wie beispielsweise Leptosphaeria nodorum;
Cercospora-Arten, wie beispielsweise Cercospora canescens;
Alternaria-Arten, wie beispielsweise Alternaria brassicae;
Pseudocercosporella-Arten, wie beispielsweise Pseudocercosporella herpotrichoides.

Die gute Pflanzenverträglichkeit der Wirkstoffe in den zur Bekämpfung von Pflanzenkranheiten notwendigen Kon zentrationen erlaubt eine Behandlung von oberirdischen Pflanzenteilen, von Pflanz-und Saatgut, und des Bodens.

Dabei lassen sich die erfindungsgemäßen Wirkstoffe mit besonders gutem Erfolg zur Bekämpfung von Getreidekrankheiten, wie beispielsweise gegen den Erreger des echten Getreidemehltaus (Erysiphe graminis), gegen den Erreger der Blattfleckenkranheit (Pyrenophora teres), gegen Cercospora-Arten und Botrytis-Arten und gegen den Erreger der Reisfleckenkrankeit (Pyricularia oryzae) einsetzen. Hervorzuheben ist, daß die erfindungsgemäßen Wirkstoffe neben einer guten protektiven Wirksamkeit auch systemische Eigenschaften besitzen. Darüberhinaus zeichnen sich die erfindungsgemäßen Wirkstoffe durch eine breite in Vitro-Wirksamkeit aus.

Die Wirkstoffe können in Abhängigkeit von ihren jeweiligen physikalischen und/oder chemischen Eigenschaften in übliche Formulierungen übergeführt werden, wie Lösungen, Emulsionen, Suspensionen, Pulver, Schäume, Pasten, Granulate, Aerosole, Wirkstoff-imprägnierte Natur-und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä., sowie ULV-Kalt-und Warmnebel-Formulierungen.

Diese Formulierungen werden in bekannter Weise hergestellt, z.B. durch Vermischen der Wirkstoffe mit Streck mitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon,

Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit so wie synthetische Granulate aus anorganischen und organischen Mehlen sowie Granulate aus organischem Material wie Sägemehl, Kokosnußschalen, Maiskolben und Tabakstengel; als Emulgier-und/oder - schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo-und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in den Formulierungen in Mischung mit anderen bekannten Wirkstoffen vorliegen wie Fungizide, Insektizide, Akarizide und Herbizide sowie in Mischungen mit Düngemitteln und Wachstumsregulatoren.

Die Wirkstoffe konnen als solche, in Form ihrer Formulierungen oder den daraus bereiteten Anwendungsformen wie gebrauchsfertige Lösungen, Suspensionen, Spritzpulver, Pasten, lösliche Pulver, Stäubemittel und Granulate angewendet werden. Die Anwendung geschieht in üblicher Weise, z.B. durch Gießen, Verspritzen, Versprühen, Verstreuen, Verstäuben, Verschäumen, Bestreichen usw.. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Bei der Behandlung von Pflanzenteilen können die Wirkstoffkonzentrationen in den Anwendungsformen in einem größeren Bereich variiert werden. Sie liegen im allgemeinen zwischen 1 und 0,0001 Gew.-%, vorzugsweise zwischen 0,5 und 0,001 %.

Bei der Saatgutbehandlung werden im allgemeinen Wirkstoffmengen von 0,001 bis 50 g je Kilogramm Saatgut, vorzugsweise 0,01 bis 10 g benötigt.

Bei Behandlung des Bodens sind Wirkstoffkonzentrationen von 0,00001 bis 0,1 Gew.-%, vorzugsweise von 0,0001 bis 0,02 % am Wirkungsort erforderlich.

Herstellungsbeispiele

Beispiel 1:

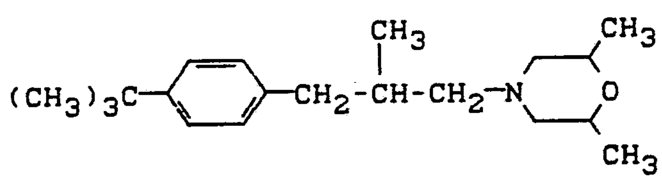

x

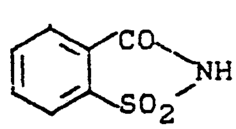

6,4 g (0,02 Mol) 3-(4-t-Butyl-phenyl)-2-methyl-1-(2,6-dimethyl-morpholin-4-yl)-propan in 50 ml Aceton werden mit einer Lösung von 3,6 g (0,02 Mol) Saccharin in 50 ml Aceton versetzt und 45 Minuten bei Raumtemperatur gerührt. Das Lösungsmittel wird im Vakuum abdestilliert und der Rückstand im Hochvakuum getrocknet.

Man erhält 10 g (100 % der Theorie) an 3-(4-t-Butyl-phenyl)-2-methyl-1-(2,6-dimethyl-morpholin-4-yl)-propan Saccharinsalz als glasartig erstarrtes zähes Öl. $^1$H-NMR ($CDCl_3$/Tetramethylsilan): $\delta$ = 7,79 (m,2H); 7,59 (m,2H); 4,07 (m,2H); 3,31 (dd,2H); 1,1 (d,3H) ppm.

In entsprechender Weise erhält man

Beispiel 2:

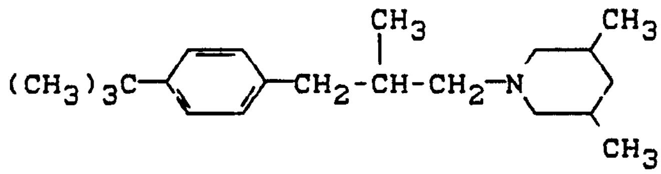

x

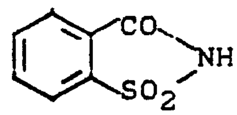

$^1$H-NMR ($CDCl_3$/TMS): $\delta$ = 3,5 - 3,6 (m,2H); 2,6 (m,2H) ppm.

Beispiel 3:

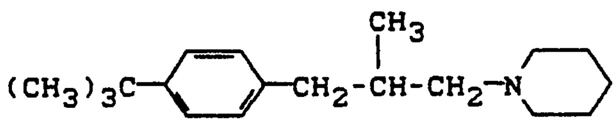

x

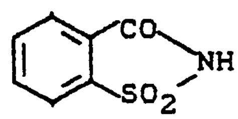

$^1$H-NMR ($CDCl_3$/TMS) : $\delta$ = 3,6 - 3,8 (m, 2H); 2,9 - 3,1 (m), 1,13 (d, 3H) ppm.

Beispiel 4:

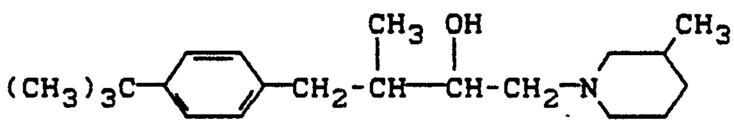

x

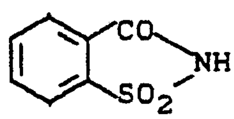

Fp: 47 °-52 °C

Beispiel 5:

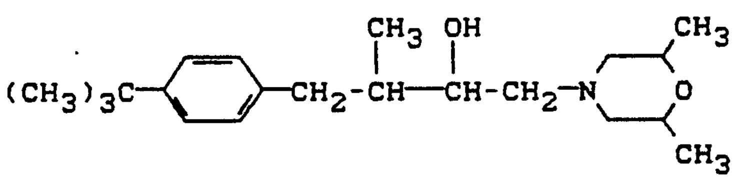

x

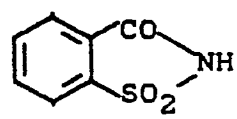

Fp: 34 ° - 38 °C

Anwendungsbeispiele:

In den folgenden Anwendungsbeispielen wurden die nachstehend aufgeführten Verbindungen als Vergleichssubstanzen eingesetzt:

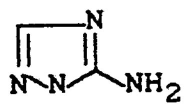 x 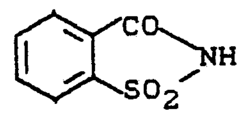 (A)

**Saccharin-Salz von 5-Amino-1,2,4-triazol**

(bekannt aus EP 158 074)
und

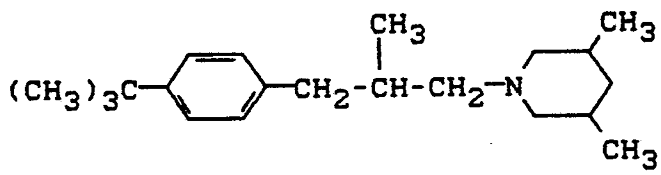 x $HCOOH$ (B)

**Ameisensäuresalz von 1-(4-t-Butylphenyl)-3-(3,5-dimethylpiperidin-1-yl)-2-methyl-propan.**

(bekannt aus DE-OS 2 752 135)

Beispiel A

Erysiphe-Test (Gerste) / protektiv

Lösungsmittel: 100 Gewichtsteile Dimethylformamid
Emulgator: 0,25 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit Sporen von Erysiphe graminis f.sp.hordei bestäubt.

Die Pflanzen werden in einem Gewächshaus bei einer Temperatur von ca. 20 °C und einer relativen Luftfeuchtigkeit von ca. 80 % aufgestellt, um die Entwicklung von Mehltaupusteln zu begünstigen.

7 Tage nach der Inokulation erfolgt die Auswertung.

Eine deutliche Überlegenheit in der Wirksamkeit gegenüber dem Stand der Technik zeigen bei diesem Test z.B. die Verbindungen gemäß den Herstellungsbeispielen: 1, 2 und 3.

Beispiel B

Pyricularia-Test (Reis) /systemisch

Lösungsmittel: 12,5 Gewichtsteile Aceton
Emulgator: 0,3 Gewichtsteile Alkylarylpolyglykolether

Zur Herstellung einer zweckmäßigen Wirkstoffzubereitung vermischt man 1 Gewichtsteil Wirkstoff mit der angegebenen Menge Lösungsmittel und verdünnt das Konzentrat mit Wasser und der angegebenen Menge Emulgator auf die gewünschte Konzentration.

Zur Prüfung auf systemische Eigenschaften werden 40 ml der Wirkstoffzubereitung auf Einheitserde gegossen, in der junge Reispflanzen angezogen wurden. 7 Tage nach der Behandlung werden die Pflanzen mit einer wäßrigen Sporensuspension von Pyricularia oryzae inokuliert. Danach verbleiben die Pflanzen in einem Gewächshaus bei einer Temperatur von 25 °C und einer rel. Luftfeuchtigkeit von 100 % bis zur Auswertung.

4 Tage nach der Inokulation erfolgt die Auswertung des Krankheitsbefalls.

Eine deutliche Überlegenheit in der Wirksamkeit gegenüber dem Stand der Technik zeigen in diesem Test z.B. die Verbindungen gemäß den Herstellungsbeispielen: 1, 2 und 3.

## Ansprüche

1. Saccharin-Salze von substituierten Aminen der allgemeinen Formel (I)

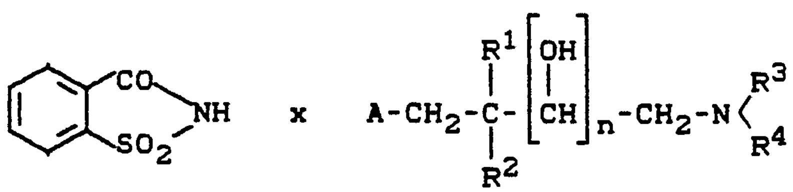

(I)

in welcher
A für jeweils gegebenenfalls substituiertes Cyclohexyl, Cyclohexenyl oder Phenyl steht,
$R^1$ für Wasserstoff oder Alkyl steht,
$R^2$ für Wasserstoff oder Alkyl steht,
$R^3$ und $R^4$ unabhängig voneinander für Alkyl stehen oder gemeinsam mit dem Stickstoffatom, an welches sie gebunden sind, für einen gegebenenfalls substituierten, gesättigten Heterocyclus stehen, der weitere Heteroatome enthalten kann und
n für eine Zahl 0 oder 1 steht.

2. Saccharin-Salze von substituierten Aminen der Formel (I) gemäß Anspruch 1, in welcher
A für jeweils ein-bis dreifach, gleich oder verschieden substituiertes Cyclohexyl oder Cyclohexenyl steht, wobei als Substituenten jeweils infrage kommen: geradkettiges oder verzweigtes Alkyl mit 1 bis 6 Kohlenstoffatomen, Halogenalkyl oder Halogenalkoxy mit jeweils 1 oder 2 Kohlenstoffatomen und 1 bis 5 gleichen oder verschiedenen Halogenatomen; oder für gegebenenfalls ein-bis dreifach, gleich oder verschieden substituiertes Phenyl steht, wobei als Substituenten infrage kommen: Halogen, geradkettiges oder verzweigtes Alkyl mit 1 bis 6 Kohlenstoffatomen, Halogenalkyl oder Halogenalkoxy mit jeweils 1 oder 2 Kohlenstoffatomen und 1 bis 5 gleichen oder verschiedenen Halogenatomen, Alkenyl mit 3 bis 6 Kohlenstoffatomen oder Cycloalkyl mit 3 bis 7 Kohlenstoffatomen;
$R^1$ für Wasserstoff oder für geradkettiges oder verzweigtes Alkyl mit 1 bis 4 Kohlenstoffatomen steht,
$R^2$ für Wasserstoff oder für geradkettiges oder verzweigtes Alkyl mit 1 bis 4 Kohlenstoffatomen steht,
$R^3$ und $R^4$ unabhängig voneinander jeweils für geradkettiges oder verzweigtes Alkyl mit 1 bis 6 Kohlenstoffatomen stehen oder gemeinsam mit dem Stickstoffatom, an welches sie gebunden sind, für einen gegebenenfalls ein-bis fünffach, gleich oder verschieden substituierten, gesättigten fünf-bis siebengliedrigen Heterocyclus stehen, der als weitere Heteroatome Stickstoff, Sauerstoff oder Schwefel enthalten kann, wobei als Substituenten infrage kommen: Hydroxy, geradkettiges oder verzweigtes Alkyl, Hydroxyalkyl, Acetoxyalkyl oder Propionyloxyalkyl mit jeweils 1 bis 4 Kohlenstoffatomen in den einzelnen Alkylteilen und
n für eine Zahl 0 oder 1 steht.

3. Saccharin-Salze von substituierten Aminen der Formel (I) gemäß Anspruch 1, in welcher
A für jeweils ein-oder zweifach, gleich oder verschieden substituiertes Cyclohexyl oder Cyclohexenyl steht, wobei als Substituenten jeweils genannt seien: Methyl, Ethyl, n-oder i-Propyl, n-, i-, s-, oder t-Butyl, 2-Methyl-but-2-yl, Trifluormethyl und Trifluormethoxy; oder für gegebenenfalls ein-bis dreifach, gleich oder verschieden substituiertes Phenyl steht, wobei als Substituenten genannt seien: Fluor, Chlor, Brom, Methyl, Ethyl, n-oder i-Propyl, n-, i-, s-oder t-Butyl, 2-Methyl-but-2-yl, Trifluormethyl, Trifluormethoxy, Allyl, n-oder i-Butenyl und Cyclohexyl;
$R^1$ für Wasserstoff oder Methyl steht,
$R^2$ für Wasserstoff oder Methyl steht,
$R^3$ und $R^4$ unabhängig voneinander jeweils für geradkettiges oder verzweigtes Alkyl mit jeweils 1 bis 6

Kohlenstoffatomen stehen, oder gemeinsam mit dem Stickstoffatom, an welches sie gebunden sind, für einen gegebenenfalls ein-bis dreifach, gleich oder verschieden durch Hydroxy, Hydroxymethyl, Methyl, Ethyl, Acetoxymethyl und Propionyloxymethyl substituierten Heterocyclus der Formel

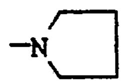 ; 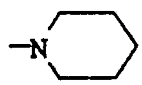 ; 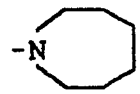 ; 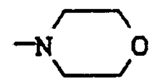 oder 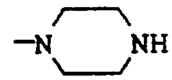

stehen und
n für eine Zahl 0 oder 1 steht.

4. Saccharin-Salze von substituierten Aminen der Formel (Ia)

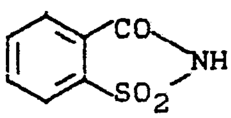 x 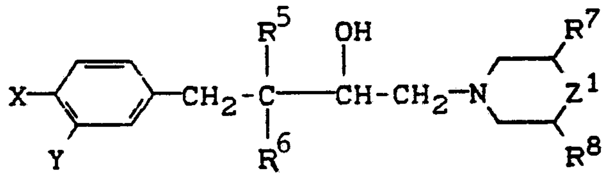 (Ia)

in welcher
X für Wasserstoff, Fluor, Chlor, i-Propyl oder t-Butyl steht,
Y für Wasserstoff, Fluor, Chlor, Methyl oder Ethyl steht,
$R^5$ für Wasserstoff oder Methyl steht,
$R^6$ für Wasserstoff oder Methyl steht,
$R^7$ und $R^8$ jeweils für Wasserstoff, Methyl oder Ethyl stehen und
$Z^1$ für Sauerstoff oder eine $-CH_2$-Gruppe steht.

5. Saccharin-Salze von substituierten Aminen der Formel (Ib)

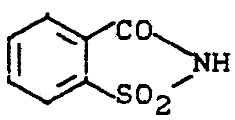 x 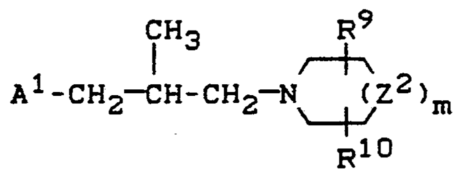 (Ib)

in welcher
$A^1$ für jeweils ein-oder zweifach, gleich oder verschieden substituiertes Cyclohexyl oder Cyclohexenyl steht, wobei als Substituenten jeweils genannt seien: Methyl, Ethyl, n-oder i-Propyl, n-, i-, s-, t-Butyl oder 2-Methyl-but-2-yl; oder für ein-oder zweifach, gleich oder verschieden substituiertes Phenyl steht, wobei als Substituenten genannt seien: Fluor, Chlor, Brom, Methyl, Ethyl, n-oder i-Propyl, n-, i-, s-oder t-Butyl, 2-Methyl-but-2-yl oder Cyclohexyl,
$R^9$ und $R^{10}$ unabhängig voneinander für Wasserstoff, Methyl, Ethyl, Hydroxymethyl oder Acetoxymethyl stehen,
$Z^2$ für Sauerstoff oder für eine gegebenenfalls durch Methyl oder Ethyl substituierte Methylen-oder Ethylenguppe steht und
m für eine Zahl 0 oder 1 steht.

6. Verfahren zur Herstellung von Saccarin-Salzen von substituierten Aminen der allgemeinen Formel (I),

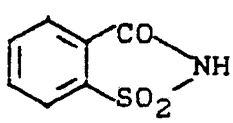 x 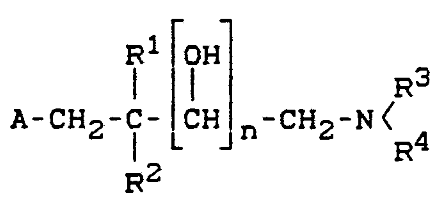 (I)

in welcher
A für jeweils gegebenenfalls substituiertes Cyclohexyl, Cyclohexenyl oder Phenyl steht,
$R^1$ für Wasserstoff oder Alkyl steht,

$R^2$ für Wasserstoff oder Alkyl steht,
$R^3$ und $R^4$ unabhängig voneinander für Alkyl stehen oder gemeinsam mit dem Stickstoffatom, an welches sie gebunden sind, für einen gegebenenfalls substituierten, gesättigten Heterocyclus stehen, der weitere Heteroatome enthalten kann und
n für eine Zahl 0 oder 1 steht,
dadurch gekennzeichnet, daß man substituierte Amine der Formel (II)

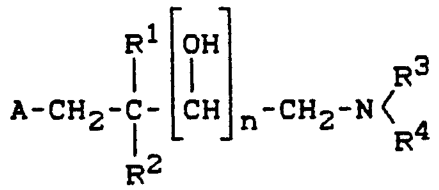 (II)

in welcher
A, $R^1$, $R^2$, $R^3$, $R^4$ und n die oben angegebene Bedeutung haben,
mit Saccharin gegebenenfalls in Gegenwart eines Verdünnungsmittels umsetzt.

7. Schädlingsbekämpfungsmittel, gekennzeichnet durch einen Gehalt an mindestens einem Saccharin-Salz von substituierten Aminen der Formeln (I), (Ia) und (Ib) gemäß den Ansprüchen 1 bis 6.

8. Verwendung von Saccharin-Salzen von Aminen der Formeln (I), (Ia) und (Ib) gemäß den Ansprüchen 1 bis 6 zur Bekämpfung von Schädlingen.

9. Verfahren zur Bekämpfung von Schädlingen, dadurch gekennzeichnet, daß man Saccharin-Salze von substituierten Aminen der Formeln (I), (Ia) und (Ib) gemäß den Ansprüchen 1 bis 6 auf Schädlinge oder ihren Lebensraum einwirken läßt.

10. Verfahren zur Herstellung von Schädlingsbekämpfungsmitteln, dadurch gekennzeichnet, daß man Saccharin-Salze von substituierten Aminen der Formeln (I), (Ia) und (Ib) gemäß den Ansprüchen 1 bis 6 mit Streckmitteln und/oder oberflächenaktiven Mitteln vermischt.

Europäisches Patentamt

# EUROPÄISCHER RECHERCHENBERICHT

Nummer der Anmeldung

EP 87 11 6506

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| D,Y | EP-A-0 158 074 (BAYER)<br>* Ansprüche *<br>--- | 1,7-10 | C 07 D 275/06<br>C 07 C 87/28<br>C 07 D 295/02<br>A 01 N 43/80 |
| D,Y | DE-A-2 752 135 (HOFFMANN-LA ROCHE)<br>* Ansprüche *<br>----- | 1,7-10 | |
| | | | **RECHERCHIERTE SACHGEBIETE (Int. Cl.4)** |
| | | | C 07 D 275/00<br>C 07 C 87/00<br>C 07 D 295/00<br>A 01 N 43/00 |

Der vorliegende Recherchenbericht wurde für alle Patentansprüche erstellt

| Recherchenort | Abschlußdatum der Recherche | Prüfer |
|---|---|---|
| DEN HAAG | 07-02-1988 | MOREAU J.M. |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)